# EUROPEAN PATENT APPLICATION

(11) **EP 4 850 664 A1**
(43) Date of publication of application: **09.12.2026**
(21) Application number: 25748478.2
(22) Date of filing: 15.01.2025
(51) Int. Cl.: A61K 8/64, A23L 33/18, A61K 38/12, A61Q 19/00

(54) **TIGHT JUNCTION RELAXATION AGENT CONTAINING SURFACTIN AND/OR SODIUM SALT THEREOF**

(30) Priority: 29.01.2024 JP 2024010915
(71) Applicant: Shiseido Company, Ltd., Chuo-ku Tokyo 104-0061 (JP)
(72) Inventor: YOSHIMURA, Mika, Tokyo 104-0061 (JP); GANTUMUR, Enkhtuul, Tokyo 104-0061 (JP)
(74) Representative: Ter Meer und Partner mbB
(86) International application number: PCT/JP2025/000937
(87) International publication number: WO 2025/164298

(57) **Abstract**

The present invention has an object of providing a novel tight junction modulator and an absorption enhancer for a compound, comprising the modulator.

The present invention provides a tight junction modulator, comprising surfactin and/or a sodium salt thereof, and an absorption enhancer for a compound, comprising such a tight junction modulator as an active ingredient.

## Description

### FIELD

The present invention relates to a tight junction modulator and an absorption enhancer for a compound, comprising the modulator.

### BACKGROUND

The skin, particularly the epidermis, is known to have a barrier function. A barrier function is a function that prevents excessive moisture transpiration from within the living body and invasion of foreign substances such as bacteria from outside the body. Such a decrease in barrier function is considered to be a cause of rough skin and dermatitis (xeroderma and atopic dermatitis).

The epidermis is the outermost membrane of the skin and has a thickness of approximately 0.1 to 0.2 mm. New cells (keratinocytes) are constantly generated in the stratum basale of the epidermis. These keratinocytes differentiate and become corneocytes through the stratum spinosum, stratum granulosum, and stratum corneum, and ultimately become skin debris and peel off. This process is called turnover. In the epidermis, turnover is repeated at a cycle of approximately one month.

During the process of turnover, a cornified envelope (hereinafter, CE), which is primarily composed of keratin fibers and wraps corneocytes, is formed. The CE is formed when a plurality of CE precursor proteins produced in keratinocytes according to differentiation of the cells are crosslinked by the enzyme transglutaminase and solubilized. Such CE is indispensable in the function of healthy stratum corneum. Specifically, formation and maturation of the CE play an important role in the improvement of skin barrier function.

In addition, liquids and substances passing through the epidermis are known to pass through two pathways: a transcellular pathway via transport proteins on the cell membrane and a paracellular pathway via intercellular gaps.

A tight junction is an intercellular adhesion mechanism (one type of intercellular binding) that seals the gaps between cells in cell sheets in the stratum granulosum. Specifically, tight junctions control the transport of liquids and substances in the paracellular pathway. Tight junctions have various skin barrier functions depending on the physiological functions of the epidermis (PTL 1). The skin barrier function of tight junctions can also be regarded as the permeability of the paracellular pathway. As such, tight junctions are particularly important in retaining moisture of the skin and in the blood-brain barrier, which prevents entry of foreign substances into the brain. In other words, when penetration of an agent into the skin to exert an effect of the agent is desired, a strong barrier function of the tight junctions acts to prevent such penetration. Medical treatments in which a high-molecular-weight functional substance such as collagen, hyaluronic acid, or Botox is injected into the skin to reduce or eliminate wrinkles or in which a laser light of a predetermined wavelength is emitted to activate metabolic functions within the dermis, thereby restoring firmness and elasticity of the skin and eliminating wrinkles, are utilized. However, the injection of such a high-molecular-weight functional substance or laser therapy is accompanied by pain during treatment, and particularly in the case of injection of a functional substance, controlling the injection depth to within approximately 2 mm in the skin is technically difficult, and there is a problem in that the final result varies depending on the skill of the practitioner. Modulating tight junctions of the skin can allow such agents that have difficulty penetrating into the skin to penetrate more easily and to exhibit the effects thereof. Accordingly, substances that modulate tight junctions have attracted attention as targets for development of cosmetics and pharmaceuticals.

### [CITATION LIST]

### [PATENT LITERATURE]

[PTL 1] Japanese Unexamined Patent Publication (Kokai) No. 2012-121871

### [NON-PATENT LITERATURE]

[NPL 1] J. Pharm. Pharmacol., 2010, 62(6), 702-708

### SUMMARY

### [TECHNICAL PROBLEM]

The present invention has an object of providing a novel tight junction modulator and an absorption enhancer for a compound, comprising the modulator.

### [SOLUTION TO PROBLEM]

The present inventors have screened various substances with the expectation of a tight junction modulating effect, and have discovered that surfactin exhibits such a tight junction modulating effect, thus completing the invention of the present application.

Accordingly, the present application encompasses the following inventions.
(1) A tight junction modulator, comprising surfactin and/or a sodium salt thereof.
(2) The tight junction modulator according to (1), which modulates epidermal tight junctions.
(3) The tight junction modulator according to (1) or (2), which modulates tight junctions via inhibition of claudin expression in cells.
(4) An absorption enhancer for a compound, comprising the tight junction modulator according to any of (1) to (3) as an active ingredient.
(5) The absorption enhancer according to (4), wherein the compound is of a medium-to-large-molecule type.
(6) The absorption enhancer according to (4) or (5), wherein the compound has a molecular weight of 4000 or greater.
(7) The absorption enhancer according to any of (4) to (6), wherein the compound is hyaluronic acid or a sodium salt thereof.

### [ADVANTAGEOUS EFFECTS OF INVENTION]

The tight junction modulator of the present invention modulates tight junctions of the skin, thereby facilitating penetration of various compounds and agents, ranging from small-molecule to large-molecule, into the skin. As a result, for example, prevention and improvement of skin conditions, such as wrinkles and sagging, and prevention and treatment of skin diseases and rough skin are achieved.

### BRIEF DESCRIPTION OF DRAWINGS

FIG. 1 shows that surfactin inhibits expression of CLDN4, thereby exhibiting claudin expression inhibitory activity and functioning as a tight junction modulator.
FIG. 2 shows that surfactin inhibits expression of CLDN7, thereby exhibiting claudin expression inhibitory activity and functioning as a tight junction modulator.
FIG. 3 shows that surfactin increases the amount of dextran permeated into skin cells.
FIG. 4 shows that surfactin increases the amount of sodium hyaluronate penetrated into the skin in a skin model.

### DESCRIPTION OF EMBODIMENTS

The present invention provides a tight junction modulator comprising surfactin and/or a sodium salt thereof.

Surfactin is known to have diverse functions such as high surfactant activity, antibacterial activity, hemolysis, and protein denaturation inhibition. Surfactin is known to be produced by microorganisms of the genus *Bacillus,* and industrial mass production of surfactin is possible by culturing the *Bacillus* microorganisms. Surfactin or a salt thereof is represented by the formula below. 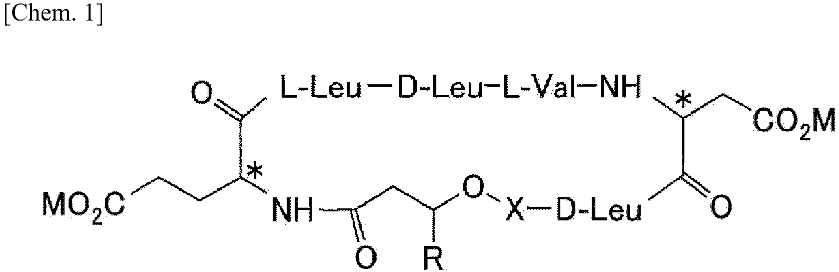 In the formula, * represents an optically active site. X represents an amino acid selected from leucine, isoleucine, and valine, and R represents an alkyl group or a branched alkyl group having 9 to 13 carbon atoms. M represents an alkali metal, an alkaline earth metal, or a substituted or unsubstituted amine. Preferably, M is sodium.

Although surfactin is known to have a surfactant function, there are no findings regarding the effect on tight junctions. Nicolo et al., J. Pharm. Pharmacol., 2010, 62(6), 702-708 (NPL 1) describes that surfactin hinders skin permeation of aciclovir.

Tight junctions are present in a belt shape around cells and serve to adhere adjacent cells to one another to seal the gaps therebetween while continuously connecting the cells. Tight junctions, which are primarily found in the stratum granulosum of the epidermis and adhere cells to one another, play an important role in preventing water and substances from permeating the intercellular gaps and maintaining barrier function. Research relating to the presence and function of proteins constituting tight junctions, such as occludin and claudins 1, 4, and 7 in the epidermis and stratum granulosum, has progressed, and more detailed mechanisms underlying skin barrier function have been elucidated. By examining the presence of or the increase in expression of these tight junction constituent proteins, such as claudins, the state of the tight junctions can also be indirectly investigated.

The tight junction modulator of the present invention, as indicated in the Examples, exhibits a tight junction modulating function by inhibiting expression of claudin-4 and/or claudin-7 in epidermal keratinocytes.

The present invention also provides an absorption enhancer for a compound, comprising the tight junction modulator of the present invention as an active ingredient, and the tight junction modulator comprising surfactin and/or a sodium salt thereof. The tight junction modulator of the present invention can be applied to the skin with a compound whose penetration into the skin is desired, specifically an agent, or can be applied to the skin in advance to modulate the tight junctions of the skin, followed by the application of another agent to the skin site, thereby enhancing penetration of the agent into the skin.

Accordingly, from another viewpoint, the present invention provides a method for allowing a compound to penetrate into the skin by modulating tight junctions of the skin, the method comprising applying the compound to the skin of a subject, to whom the penetration of the compound into the skin is desired, together with the tight junction modulator of the present invention, or applying the tight junction modulator of the present invention to the skin in advance to modulate the tight junctions of the skin and then applying the compound to the skin site.

Additional compounds or agents whose penetration is desired are not particularly limited, and need only be pharmaceutically or cosmetically acceptable, from small-molecule compounds, to medium-to-large-molecule compounds such as polysaccharides, peptides, and proteins. Examples of pharmaceutical agents include antiviral agents, antifungal agents, antibiotics, growth factors, and drugs. Examples of cosmetically active agents include cosmetic agents, for example, small-molecule compounds such as vitamin C, vitamin C derivatives, vitamin A derivatives, vitamin E derivatives, inositol, xylitol, glycerin, tranexamic acid, niacinamide, glycylglycine, 4-methoxysalicylate, salicylic acid, hydroxyethyl imidazolidinone, amino acids, and urea; medium-to-large-molecule compounds such as hyaluronic acid and sodium salts thereof, glucosamine, elastin, collagen, polylactic acid, and dextrin; and large-molecule compounds such as Botox.

In the present specification, small-molecule compound refers to, for example, a compound having a molecular weight of less than 1,000; medium-molecule compound refers to, for example, a compound having a molecular weight of 1,000 or greater and 10,000 or less; and large-molecule compound refers to, for example, a compound having a molecular weight of greater than 10,000. Therefore, medium-to-large-molecule may refer to a compound having a molecular weight of, for example, 1,000 or greater, 2,000 or greater, 3,000 or greater, or 4,000 or greater, and 4,000,000 or less, 3,000,000 or less, or 2,000,000 or less.

In the present invention, the additional compound whose penetration into the skin is desired may be an extract, and for example, may be yeast extract, cherry leaf extract, cinnamon bark extract, pine extract, Bulgarian rose water, okra extract, Japanese thyme extract, burnet extract, dipotassium glycyrrhizate, ginkgo leaf extract, *Rubus suavissimus* (raspberry) leaf extract, Job's tears extract, licorice extract, ginseng extract, shrubby sophora extract, starfruit leaf extract, carrot extract, houttuynia extract, neem leaf extract, saffron extract, cinchona extract, comfrey extract, thyme extract, *Rubus buergeri*, Japanese aster, stauntonia vine, Ryukyu strawberry, Indian aster, Japanese stone oak, Baikal skullcap root extract, rosemary extract, apricot kernel, gentian extract, peppermint powder, jujube extract, hops extract, kiwi extract, Roman chamomile extract, apple extract, Japanese hawthorn extract, turmeric extract, red bayberry, rose apple, and jolcham oak. These extracts can be used independently or in combination of a plurality thereof.

In the present invention, a particularly preferable agent for penetration is hyaluronic acid or a sodium salt thereof. Hyaluronic acid or a sodium salt thereof has many functions, such as retention of moisture in intercellular gaps, retention of cells based on formation of a jelly-like matrix within tissues, maintenance of tissue lubricity and flexibility, resistance against external forces such as mechanical damage, and prevention of bacterial infection. It is said that, for example, the amount of hyaluronic acid or a sodium salt thereof in the skin decreases with age, and signs of skin aging such as fine wrinkles and dryness appear as a result. Therefore, many cosmetics in which hyaluronic acid or a sodium salt thereof is blended have been proposed as agents for improving such aging skin. The molecular weight of hyaluronic acid or a sodium salt thereof ranges widely, and when hydrolyzed by protease, the lower limit may be approximately 1,500 to 5,000 in molecular weight, and the upper limit may reach 800,000 to 4,000,000. In the present invention, it is considered that hyaluronic acid or a sodium salt thereof having a molecular weight of at least 4,000 and at most 2,000,000, or at most approximately 4,000,000, can penetrate into the skin.

The tight junction modulator of the present invention can refer to, for example, the skin penetration of an additional compound, whose penetration into the skin is desired, being increased by, for example, 10% or greater, 20% or greater, 30% or greater, 40% or greater, 50% or greater, 60% or greater, 70% or greater, 80% or greater, 90% or greater, 100% or greater, 200% or greater, 300% or greater, 400% or greater, or 500% or greater, compared to the absence of the tight junction modulator of the present invention.

The tight junction modulator of the present invention can be used in drug absorption aids, pharmaceutical compositions, quasi-drugs, and cosmetics. Examples of dosage forms thereof include film, powder, pill, tablet, injection, suppository, emulsion, capsule, granule, liquid (including tincture, fluid extract, spirit, suspension, and lemonade). Various components used in conventional quasi-drugs, pharmaceuticals, and cosmetics, for example, oil-based components, emulsifiers, moisturizers, thickeners, medicinal components, preservatives, powders, pH adjusters, ultraviolet absorbers, and antioxidants can be appropriately blended in ranges that do not impair the effect of the present invention.

All references mentioned in the present specification are incorporated into the present specification in their entirety by citation.

The Examples of the present invention described below are provided for illustrative purposes only, and do not limit the technical scope of the present invention. The technical scope of the present invention is limited only by the claims. Modifications of the present invention, for example, additions, deletions, and substitutions of constituent elements of the present invention can be made without departing from the spirit of the present invention.

### [EXAMPLE 1]

Hereinafter, the present invention will be further described in detail based on the Examples. However, the present invention is not limited thereto in any way.

### Experiment 1: Effects of various compounds on expression of tight junction constituent proteins Experimental method: Western blotting

In a 6-well plate, normal human epidermal keratinocytes (NHEK) were seeded in KGM-Gold(trademark) (Lonza Verviers, Belgium) medium, to which supplements were added, and incubated for 9 d. The medium was then changed to a medium adjusted to a calcium concentration of 1.3 mM, and the cells were cultured for 4 d, followed by culturing in a mixed medium, to which 1 ng/mL of a growth promoting factor was added, for 16 d. A medium containing various compounds (medium adjusted to a calcium concentration of 1.3 mM + 1 ng/mL of growth promoting factor) was then added, and the cells were treated for 24 h. After treatment, the cells were washed twice with serum-free α-MEM medium. A sample buffer for electrophoresis was added, and the cells were collected using a cell scraper. The sample was sonicated, subjected to SDS-PAGE followed by Western blotting. Tight junction constituent proteins claudin-4 (CLDN4) and claudin-7 (CLDN7) were detected with antibodies thereof. β-Actin was used as the internal standard protein.

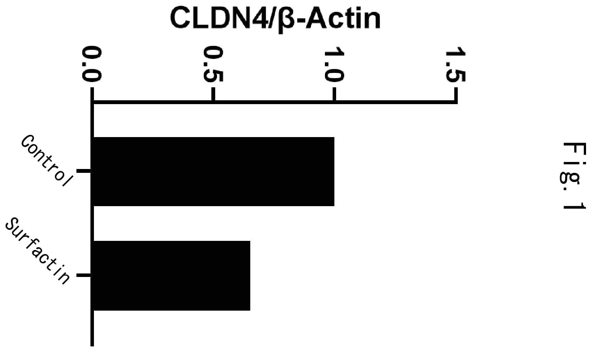

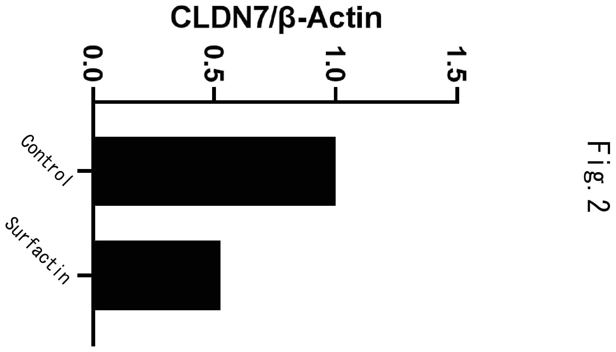

Results thereof are shown in FIG. 1 and FIG. 2. It was suggested that surfactin, by inhibiting the expression of CLDN4 and CLDN7, exhibited claudin expression inhibitory activity and functioned as a tight junction modulator.

### Experiment 2: Effects of various compounds on barrier function

### Experimental method: Permeation assay

In the upper chamber of a collagen-coated 24-transwell plate, normal human epidermal keratinocytes (NHEK) were seeded in KGM-Gold(trademark) (Lonza Verviers, Belgium) medium, to which supplements were added, and incubated for 11 d. The medium was then changed to a medium adjusted to a calcium concentration of 1.3 mM, and the cells were cultured for 2 d. The medium in the upper chamber was then removed and dried, and the cells were cultured for 12 d, while a mixed medium in the lower chamber was replaced once every 2 d. Subsequently, 100 µL of HBSS (Hank's Balanced Salt Solution) containing various compounds and 2 µM FITC-dextran was added to the upper chamber, and the cells were left to stand in an incubator for 3 h, after which the HBSS was removed. After 24 h, 100 µL of the medium in the lower chamber was sampled, and fluorescence intensity of FITC was measured by a fluorescence plate reader.

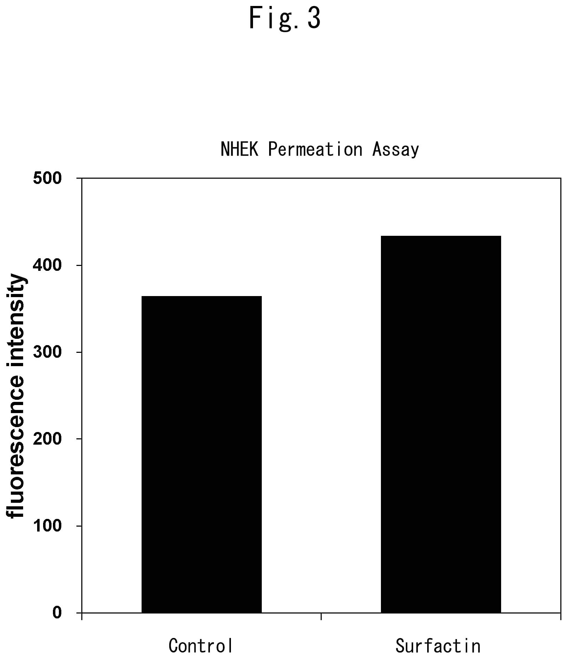

Results thereof are shown in FIG. 3. Surfactin, which has been suggested to function as a tight junction modulator, was shown to have increased the amount of dextran permeated, compared to the control (medium). Therefore, it was shown that surfactin functioned as a tight junction modulator and lowered epidermal barrier function.

### Experiment 3: Effect of surfactin on penetration of sodium hyaluronate

Using EPI-200X (Kurabo) as the skin model, the effect of surfactin on penetration of sodium hyaluronate was examined. Specifically,
1. As a receiver solution, 0.9 mL of Dulbecco's phosphate buffered saline (D-PBS) warmed to 37°C or D-PBS in which 0.005% surfactin was dissolved was added to a 6-well plate.
2. EpiDerm EPI-200X (Kurabo Industries Ltd.; hereinafter, skin model) was set in a diffusion chamber (EPI-FIX, KURABO) and set in the 6-well plate prepared in step 1. The assembly was equilibrated in a CO2 incubator for 15 min.
3. 0.35 mL of an aqueous solution of 0.1% fluoresceinamine-labeled sodium hyaluronate (average molecular weight of 1,200,000 to 1,600,000, FAHA-H2, PG Research; hereinafter, FA-HA) was added to each donor.
4. The assembly was incubated in a CO2 incubator. After 24 h, the skin model was collected.
5. The skin model surface was thoroughly washed with MQ water, sectioned with a scalpel, and placed in an Eppendorf tube.
6. The skin model was frozen in liquid nitrogen, followed by 30 s of pulverization, and D-PBS was added. The mixture was subjected to ultrasonic treatment for 15 min and then centrifuged (100 rpm) for 20 min. The resulting supernatant was transferred to another Eppendorf tube.
7. Measurement was carried out using a microplate reader (excitation wavelength of 490 nm, fluorescence wavelength of 520 nm).

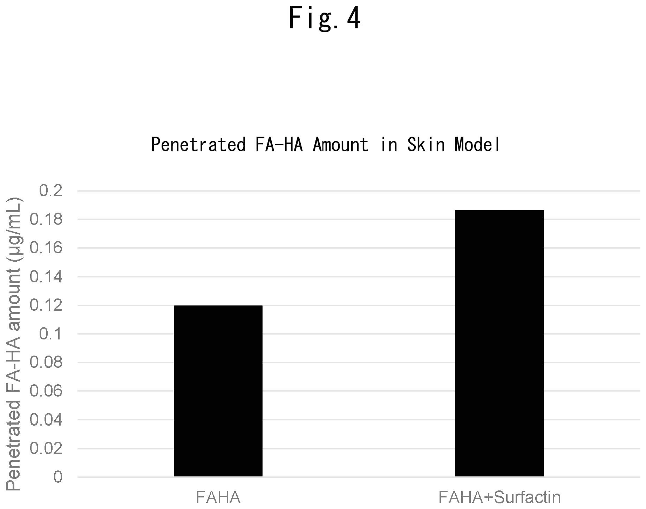

Results thereof are shown in FIG. 4. Surfactin, which functioned as a tight junction modulator and lowered epidermal barrier function, was shown to have increased the amount of sodium hyaluronate penetrated into the skin.

### INDUSTRIAL APPLICABILITY

Surfactin and/or a sodium salt thereof can modulate tight junctions. Accordingly, surfactin and/or a sodium salt thereof can be used as absorption enhancers for compounds and as pharmaceutical compositions, quasi-drug compositions, cosmetic compositions, and food compositions, and are therefore useful for the medical, cosmetic, and food industries.

## Claims

1. A tight junction modulator, comprising surfactin and/or a sodium salt thereof.

2. The tight junction modulator according to claim 1, which modulates epidermal tight junctions.

3. The tight junction modulator according to claim 1 or 2, which modulates tight junctions via inhibition of claudin expression in cells.

4. An absorption enhancer for a compound, comprising the tight junction modulator according to any one of claims 1 to 3 as an active ingredient.

5. The absorption enhancer according to claim 4, wherein the compound is of a medium-to-large-molecule type.

6. The absorption enhancer according to claim 4 or 5, wherein the compound has a molecular weight of 4000 or greater.

7. The absorption enhancer according to any one of claims 4 to 6, wherein the compound is hyaluronic acid or a sodium salt thereof.
